# EUROPEAN PATENT APPLICATION

(11) **EP 4 516 285 A1**
(43) Date of publication of application: **05.03.2025**
(21) Application number: 24197917.8
(22) Date of filing: 02.09.2024
(51) Int. Cl.: A61K 8/9789, A61K 8/73, A61P 17/06, A61P 17/08, A61P 17/10, A61P 17/14, A61P 17/16, A61Q 19/00, A61Q 19/08, A61K 36/73

(54) **EXTRACT ENRICHED IN UNSATURATED FATTY ACIDS FROM THE PROCESSING WASTE OF FOOD PRODUCTS BASED ON WILD STRAWBERRIES AND/OR GARDEN STRAWBERRIES**

(30) Priority: 04.09.2023 IT 202300018183
(71) Applicant: Unifarco S.p.A., 32035 Santa Giustina (BL) (IT)
(72) Inventor: Baratto, Giovanni, 32035 Santa Giustina BL (IT); De Nadai, Gabriele, 32035 Santa Giustina BL (IT); Ferrari, Sara, 32035 Santa Giustina BL (IT); Francescato, Stefano, 32035 Santa Giustina BL (IT)
(74) Representative: Perani & Partners S.p.A.

(57) **Abstract**

Extract enriched in unsaturated fatty acids from processing waste in the production of foods based on *Fragaria vesca* (wild strawberry) and/or *Fragaria x ananassa* (strawberry or garden strawberry) obtained by supercritical extraction in CO2, characterised by the fact that it has a peroxide content less than 60 meq/kg of extract, related preparation process, related compositions in particular for cosmetic use.

This extract can be used for the treatment and prevention of disorders of inflammatory origin, such as atopic dermatitis and psoriasis, and/or it can be used for the treatment of skin and hair disorders associated with hormonal imbalance, for example overproduction of dihydrotestosterone, such as acne vulgaris, hirsutism, seborrhoea, telogen effluvium and androgenic alopecia, or it can be used as an emollient and/or humectant to restore the moisture of the barrier layer.

## Description

### FIELD OF THE INVENTION

The present invention relates to an extract enriched in fatty acids, the related preparation process, in particular cosmetic compositions containing it, as well as the related use in the treatment and prevention of skin and hair disorders of inflammatory origin and/or associated with hormonal imbalance.

### BACKGROUND OF THE INVENTION

In recent years, due to dramatic climate change, sustainability is a major challenge which companies must consider during the entire product development process, from the selection of raw materials up to the production process in order to minimize environmental impact at every stage. Many raw materials obtained from organic waste are spreading in the world of cosmetics, and it has become necessary to source new raw materials with low environmental impact, such as "upcycled" ingredients from short and circular supply chains.

Above all, agri-food waste is now a very attractive starting point for producing innovative and sustainable cosmetic extracts. The peels and seeds of fruit, for example, are very rich in phytochemicals, with multiple application possibilities in cosmetics, such as antioxidant polyphenols or anti-inflammatory fatty acids. [1,2,3]

With the aim of recovering interesting active ingredients from food waste, the applicant has created an upcycling chain in collaboration with a local company, to produce a cosmetic ingredient by reusing fruit processing waste through an eco-compatible extraction process in supercritical CO2 (sCO2).

The selection of certified organic biomass guarantees the quality of the material and ensures the absence of contaminants allowed in conventional agriculture, such as pesticides. We have previously studied biomass extracts of different fruits containing skins and seeds (pomace) of raspberry, blueberry, wild strawberry, pomegranate, blackberry and black currants derived from organic farming. [3]

Supercritical fluid extraction based on carbon dioxide (sCO2) has been previously identified as the most effective method for extracting these lipids from the pomace of the berries. [3] Previous results have shown the oily extract from the peel and seeds of wild strawberries as the most promising for its qualitative-quantitative phytochemical composition. [3] *Fragaria vesca* L. (Wild strawberry) is a perennial herbaceous plant of the Rosaceae family which produces edible red fruits and grows naturally throughout Northern Europe. Wild strawberries can be cultivated and harvested for domestic use and for the industrial production of food products such as jams and liqueurs [4]. Its seeds contain an oil rich in unsaturated fatty acids (e.g., linoleic and linolenic acid) of interest for cosmetic applications. [1]

Unsaturated fatty acids are essential macronutrients which are attracting attention as potential agents for maintaining skin health and treating skin disorders, particularly those mediated by solar ultraviolet radiation (UVR), including sunburn, cancer, photosensitivity and photo-ageing. [5] Indeed, it appears that long-chain polyunsaturated fatty acids have a potential to decrease inflammatory processes, which could be useful for the management of inflammatory diseases of the skin, such as atopic dermatitis, psoriasis and acne [2]. As a source of the main lipid components of the stratum corneum, the extract obtained could be used as a valuable moisturising agent for cosmetic products, especially as an emollient and to prevent skin dehydration. [1]

It is also reported in the literature that unsaturated fatty acids can inhibit 5α-reductase, the enzyme which catalyses the conversion of testosterone to its most functionally active metabolite, dihydrotestosterone. [6] 5α-reductase is expressed in skin melanocytes, fibroblasts, and keratinocytes. [7,8] This hormone is implicated in various skin disorders such as acne vulgaris, hirsutism, seborrhoea, *telogen effluvium,* and androgenetic alopecia. [7,8] After birth, type 1 5α-reductase is expressed in multiple tissues; including the liver, skin, scalp, and prostate, while type 2 5α-reductase is expressed in the prostate, seminal vesicles, epididymis, liver, and to a lesser extent, in the scalp and skin. [8, 9,10]

In an attempt to produce a raw material of such quality that it can be included as an ingredient in a formulation for cosmetic or nutraceutical use, the applicant has identified an unresolved problem with this extract, namely the presence of a high peroxide content.

Peroxides are precursors to decomposition products which can cause a rancid taste in fats. Peroxide levels are an indicator of oxidation in the early stages of lipid degradation. The index is less reliable in the last stage of the process, when lipid degradation increases.

The amount of peroxide (POV) refers to the reactive oxygen content expressed in milliequivalents (meq) of free iodine per kilogram of fat. It is measured by titrating the iodine dissolved by potassium iodide using sodium thiosulphate as a solution. Oils which have a POV of less than 10 meq/kg are considered fresh. The rancid taste is evident when the POV rises between 40 and 60 meq/kg. When interpreting these figures, however, it is important to be aware of the specific fat or oil involved.

KR101099664B1 discloses a cosmetic composition for the treatment of skin disorders caused by an overproduction of dihydrotestosterone by the enzyme 5-alpha-reductase by an extract of strawberry sepal, preferably of the species *Fragaria* x *ananassa.* Although supercritical CO2 extraction is envisaged for preparing the extract, the only exemplified type envisages extraction in water or a water-soluble solvent such as ethanol or diols.

### SUMMARY OF THE INVENTION.

The applicant has now found an extract enriched in unsaturated fatty acids from processing waste in the production of foods based on *Fragaria vesca* and/or *Fragaria* x *ananassa* which is characterised in that it has a quantity of peroxides less than 40 meq/kg obtained by supercritical CO2 extraction.

This extract is prepared with a process which envisages that before the extraction with supercritical CO2, the waste product from the processing of *Fragaria vesca* and *Fragaria* x *ananassa* is dried at a temperature comprised between 25 and 45°C for a time comprised between 48 and 120 hours in an ionizing environment.

A further object of the present invention is in particular cosmetic compositions containing the extract subject matter of the present invention.

A further object of the present invention is the use of the extract subject matter of the present invention for the treatment of skin and hair disorders of anti-inflammatory origin and/or associated with hormonal imbalance.

A further object of the invention is the use of said extract in the cosmetic field as an emollient and humectant for maintaining the moisture of the skin barrier layer.

The extract subject matter of the present invention preferably contains between 60 and 90% of unsaturated fatty acids, more preferably between 65 and 85% by weight, on the total weight of the extract.

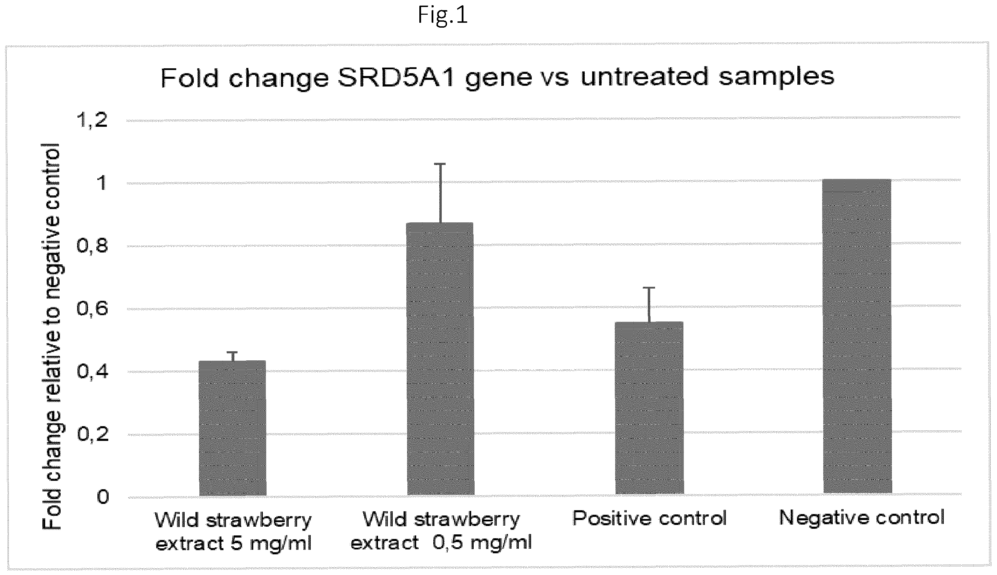

### DESCRIPTION OF FIGURE 1

Figure 1 shows in the graph the fold change of the SRDA51 gene expression of the samples treated with the extract according to the present invention vs the negative control (untreated samples)

### DETAILED DISCLOSURE OF THE INVENTION.

For the purposes of the present invention, the definitions "comprising, containing" do not exclude the presence of additional components/stages in addition to those listed after such definitions. The definition "consisting of, constituted by" excludes the presence of further components/stages in addition to those expressly listed after such definitions.

The term *Fragaria vesca* refers to wild forest strawberry grown naturally, while the term *Fragaria x ananassa* refers to what is known as the strawberry or garden strawberry, in other words a hybrid suitable for cultivation.

For the purposes of the present invention, processing waste of wild forest strawberry and garden strawberry contained in the extract subject matter of the present invention, is intended as that coming from the production of jams, jellies, purées, juices or other food derivatives.

It comprises seeds, skin and pulp residues, preferably it consists of seeds, skin and pulp residues of wild strawberries and garden strawberries.

Preferably the quantity of peroxides is less than 40 meq/ kg of extract, more preferably it is comprised between 15 and 35 meq/kg, even more preferably between 25 and 30 meq/kg of extract. The process for preparing the extract subject matter of the present invention comprises the following steps:
a) said food processing waste based on *Fragaria vesca* and/or *Fragaria x ananassa* is dried in an ionizing environment at a temperature comprised between 25° and 45°C for a time comprised between 48 and 120 hours;
b) the dried product from step a) is supercritically extracted with CO2.

According to a particularly preferred form of the invention, step a) is performed according to the following operating modes: the material is dried in ionizing environment preferably frozen at a temperature comprised between 30 and 40°C for 48 hours and at 45°C for 72 hours.

The ionizing environment is obtained with an ionizer or ionizing lamp.

Step b) is instead carried out using operating conditions of a conventional type and known to the person skilled in the art.

Preferably, the cosmetic compositions subject matter of the invention are preferably administrable topically and are of a conventional type such as ointments, oils, creams, oil-in-water or water-in-oil emulsions, etc.

They contain the extract in concentrations comprised between 0.1 and 2%, preferably between 0.5 and 1% by weight.

As anticipated above, the extract subject matter of the invention can be advantageously used to treat or prevent skin disorders of inflammatory origin such as: atopic dermatitis, psoriasis, sunburn, skin cancer, skin ageing, photosensitivity and photo ageing.

The hormonal imbalance which can cause skin disorders consists essentially of the overproduction of dihydrotestosterone by the enzyme 5-α-reductase present in skin cells such as fibroblasts, keratinocytes etc. The excess production of dihydrotestosterone can be advantageously counteracted thanks to the extract according to the present invention as reported in the experimental tests reported below.

Therefore, the extract subject matter of the invention can be advantageously used for the prevention and treatment of *acne vulgaris,* hirsutism, seborrhoea, *telogen effluvium* and androgenic alopecia.

The extract subject matter of the present invention as underlined can be used in cosmetic compositions as a humectant and/or emollient to restore the moisture of the skin barrier layer.

The preparation examples of the extracts subject matter of the present invention and the efficacy tests in inhibiting 5-α reductase type 2, the stability tests and the composition of such extracts and the comparison of peroxide content obtained with the sCO2 extraction process of the prior art described in [3], which does not envisage the specific drying of step a) of the process of the invention, are given merely for illustrative purposes.

### EXAMPLE 1

### 1-A Materials and methods

### 1-A-1 Equipment and chemical compounds

All the chemical compounds and solvents are of a commercial type and have not undergone further purification. The supercritical CO2 extraction was performed in a laboratory apparatus consisting of a Jasco Plus Delivery PU-2080-CO2 pump and a Jasco 2080 Plus Automatic back pressure regulator used for pressure control. The stainless steel connection pipes and the different reactors were heated with a Chrompack CP-9003 oven. The GC-MS analysis was carried out using an Agilent 7820A GC system coupled with an Agilent 5977B MSD single quadrupole mass detector equipped with an HP88 column (60m x0.25 mm, 0.2 µm).

### 1-A-2 Sample preparation

The frozen waste material (seeds and skins) of wild strawberry was supplied by Rigoni di Asiago (Asiago (VI)). The frozen sample was weighed before and after drying to determine the water content in the fresh biomass. The biomass was subjected to drying in an air oven at 40°C for 48 hours. The loss on drying (LOD) is the resulting w/w percentage of the sample after evaporation of water and other volatile substances from the dried sample. The LOD measurements of fresh and dried product were measured at 105°C for 15 minutes.

### 1-A-3 Supercritical fluid extraction

The experimental extraction tests were carried out on an analytical scale extractive unit (internal volume of approximately 10 cm³, diameter 3/8", length 15 cm) using sCO2 as solvent. The extraction vessel was loaded with approximately 3 g (pre-pilot batch) and 1 kg (pilot batch) of dry pomace ground at a constant flow rate of supercritical CO2 (5.0 or 2.5 cm3 min-1) at 300 bar, 70 °C for 5 h. The extracts were collected by hexane ventilation at room temperature and pressure and were gravimetrically quantified (extract yield = extract weight/dry biomass weight %) at the end of each run (after hexane removal). GC-MS columns employing methyl pentadecanoate (2 mg/mL) as an internal standard were used for the quantitative analysis of the extract.

### 1-A-4 Chromatographic analysis of the extracts

Before carrying out the GC-MS analysis, the fatty acids (FAs) contained in the extracts were converted to the corresponding methyl esters (FAMEs) by dissolving the extracts (50 mg exactly weighed) in chloroform (5 mL) followed by the addition of methanol (25 mL), internal standard (methyl pentadecanoate 0.5 mL chloroform solution 500 µg/mL) and a catalytic amount of sulphuric acid. The resulting solution was refluxed overnight. The FAMEs were extracted with a mixture of water and diethyl ether (10:2). The organic fraction was analysed by means of GC-MS. The fatty acids were identified by comparing the MS spectra and compound retention index. The quantification was obtained using methyl pentadecanoate as internal standard.

### 1-A-5 Efficacy tests

The modulation of the 5α-reductase gene expression in cultured cells exposed to different concentrations of the test substance compared to untreated cells was evaluated.

The test was carried out on primary fibroblasts. The cell culture was pre-treated with testosterone to induce the overproduction of endogenous 5α-reductase before being exposed to the tested substances.

A titrated extract of *Serenoa repens* (Saw palmetto), a well-known pharmacologically active compound used to treat prostatic hyperplasia, acts as the positive control. Gene expression was assessed by means of qRT-PCR, a sensitive and reliable method for the detection and quantification of specific nucleic acid level sequences. qRT-PCR measures gene expression by quantifying the fluorescence emitted during the reaction as an indicator of amplicon synthesis over the course of the cycle. [11]

The cells were seeded in 6-well plates and left to grow for 24 hours. After that, the cultures were treated for 24 hours with 10 ng/ml testosterone. After this incubation, a fresh medium was added, added with the tested sample at subtoxic concentrations (5 and 0.5 mg/ml). The sample was dissolved in ethanol, then diluted in the culture medium. The concentrations were defined based on preliminary cytotoxicity assay.

The untreated cells were used as a negative control, while the cells treated with Saw palmetto extract (10 µg/ml) were used as a positive control.

Each sample was treated in triplicate. After 48 hours of exposure, the total RNA purified from the cells is dissolved in 50 µl of purified sterile water and its concentration was determined by means of spectrophotometric reading.

Changes in the gene expression profile were analysed with the Real Time PCR technique, using the TaqMan assay. The primer pair sequence of the 5-alpha reductase 1 gene (SRD5A1 gene) was designed within the exons. The TaqMan probe principle is based on the activity of the Taq polymerase 5'-3' exonuclease to cleave the dual-labelled probe during hybridization to the complementary target sequence and fluorophore-based detection. As in other quantitative PCR methods, the resulting fluorescence signal allows quantitative measurements of product accumulation during the exponential stages of the PCR. [12].

### 1-A-6 Inclusion of the extracts in cosmetic products and stability tests

The sCO2 extracts were added at 0.5% and 1% in an oily matrix to evaluate the stability and relative performance in the final formulation. The samples were tested at different temperatures (5°C, 40°C, 50°C in the oven in a dry state) and room temperature exposed to light. The centrifugation and secondary analysis tests were carried out under all the conditions at two different time points (Time zero (T0) and after 1 month).

### 1-B- Results

### 1-B-1 Pretreatment of the biomass

The biomass pre-treatment results are shown in Table 1. The water content in the fresh biomass pomace (44.1%) is the weight difference between the initial weight and the dry air-dried sample. The LOD of the fresh biomass is greater than 50% and after 48 hours is 3.6%.

**Table 1**

| Wild strawberry waste biomass | |
|---|---|
| water (w%) | 44.1 |
| Loss on drying (LOD) of the fresh biomass | 52.1 |
| Loss on drying (LOD) of the dry biomass | 3.6 |

### 1-B-2 Phytochemical characterisation of the fatty acids in the extract

The extraction yield is 13.5% for the pre-pilot batch and 11% for the pilot batch, which confirms that these biomasses have a good content of lipophilic compounds extractable with sCO2.

The quantitative analysis of fatty acids is reported in Table 2.

From the results summarised therein, it is observed that the "pre-pilot batch" extraction leads to an extract richer in fatty acids with respect to the "pilot batch", the variability is however moderate and could be due to the different composition of the plant material from which it is derived. 15 different fatty acids were identified, including linoleic and linolenic (unsaturated) as main components. In the pre-pilot batch, the total weight of unsaturated fatty acids is 84%, while in the pilot batch it is 69%. The saturated fatty acid content is very similar for the pre-pilot and the pilot batch and it is about 7.5%. Most of the unsaturated fatty acids found in the s-CO2 extracts are n-6, 74% and 57% in the pre-pilot and pilot batch, respectively.

**Table 2 characterisation and GC retention times of fatty acids in wild strawberry.**

| Retention time [min] | Abbreviation | Fatty acid | Pre-pilot batch fatty acid w/w [%] | Pilot batch fatty acid w/w[%] |
|---|---|---|---|---|
| 14.51 | C12:0 | lauric acid | 0.182 | 0.227 |
| 19.18 | C14:0 | myristic acid | 0.103 | 0.164 |
| 22.92 | C16:0 | palmitic acid | 4.458 | 3.768 |
| 24.23 | C16:1 | Palmitoleic Acid | 0.123 | 0.247 |
| 25.14 | C17:0 | Heptadecanoic acid | 0.16 | ND |
| 27.37 | C18:0 | Stearic acid | 1.956 | 2.421 |
| 28.45 | C18:1 cis (n9) | Oleic acid | 9.193 | 10.717 |
| 28.6 | C18:1 trans (n9) | Elaidic acid | 0.508 | 0.897 |
| 30.18 | C18:2 cis (n6) | Linoleic acid | 36.328 | 25.595 |
| 31.5 | C20:0 | Arachidic acid | 0.786 | 0.921 |
| 32.15 | C18:3 (n6) | Linolenic acid | 37.84 | 31.245 |
| 32.53 | C20:1 (n9) | cis-11 eicosenoic acid | 0.298 | 0.474 |
| 34.17 | C20:2 (n6) | cis-11,14 eicosadienoic acid | 0.19 | 0.314 |
| 36.25 | C22:0 | behenic acid | ND | ND |

### 1-B-3 -Efficacy tests

The sample is able to modulate the gene expression levels of 5-alpha reductase type I (SRD5A1) after 48 hours of treatment, at a concentration of 5 mg/ml. In the cells treated with the sample, the gene expression is lower than that measured in the untreated cells, i.e., the negative control (NC), whose levels have been arbitrarily assigned a value of 1.

The data reported suggest that under the aforesaid experimental conditions, the sample is capable of inhibiting expression levels of SRD5A1.

The fold changes in the gene expression of 5 alpha reductase type I are reported in the following Table 3 and in Fig. 1.

**Table 3-Fold change of the SRD5A1 gene (±sd) vs untreated samples**

| | |
|---|---|
| Wild strawberry waste sCO2 extract (Batch 14062021) 5 mg/ml | 0.43(0.03); *p value*=*0.035* |
| Wild strawberry waste sCO2 extract (Batch 14062021) 0.5 mg/ml | 0.87 (0.19); *p value=NS* |
| Saw palmetto 10µg/ml (Positive control) | 0.55 (0.11); *p value* =*0.036* |

### 1-B-4 Inclusion of the extracts according to the present invention in cosmetic products and related stability test.

The stability conditions at time T0 of the body oils containing the sCO2 extract according to the present invention were analysed by centrifugation and pH measurement.

Both prototypes (pilot and pre-pilot sCO2 extract) remained stable at different conditions (5°C, 40°C, 50°C in the oven) at time T0 and after 1 month, no obvious change in colour and odour was observed and the oils did not show separation or precipitate formation both during formulation (T0), and after 1 month.

### EXAMPLE 2

From 2 batches of biomass (vegetable raw material batch no. 22092022B-extract batch E1301D and vegetable raw material batch no. 22092022A- extract batch E1302D) consisting of 100% waste (seeds, skin and pulp residues) of *Fragaria x ananassa,* weighing 21 kg and 7 kg respectively, 1122 g (extract batch E1302D) and 434 g (extract batch E1301D) of extract respectively are obtained according to the present invention and prepared with the process of the invention wherein step a) envisages the following operating conditions: drying at a temperature comprised between 30 and 40°C for 48 hours and at 45°C for 72 hours in the presence of an ionizing lamp. The extracts of the two batches were then titrated as described to verify the quantity of peroxides. For the first batch the quantity of peroxides was equal to 27.6 meq/kg, while for the second batch it was equal to 26.1 meq/kg of extract.

### EXAMPLE 2A

From the biomass batch SFR00122/A consisting of 100% *Fragaria x ananassa* waste weighing 5.666 kg, 300 g were extracted according to the procedure described in [3], wherein step a) envisages that the starting biomass is subjected to air drying at a temperature above 40°C and uncontrolled for 48 hours. The extract was titrated to evaluate the peroxide content, which gave the following value 141.4 meq/kg; therefore, it has a peroxide content at least 5 times that contained in the extracts subject matter of the invention and prepared as described in Example 2.

### EXAMPLE 3

Two extract batches (E1301D and E1302D) were obtained from 2 biomass batches (22092022 B and 22092022 A). The extracts were prepared according to the procedure described in Example 2. Both extracts obtained were titrated and gave as a quantity in peroxides 27.6 and 26.1 meq/kg. Two sub-batches were created from these two batches of extract, adding two antioxidants:
1) E1301D/A consisting of 99.8% oil extracted from *Fragaria x ananassa* and 0.2% antioxidant extract of rosemary (for example, the raw material *Rosmarinus Officinalis* Leaf Extract, CAS-No. 84604-14-8, EINECS-No. 283-291-9 dissolved in *Helianthus Annuus* Seed Oil, CAS-No. 8001-21-6, EINECS-No. 232-273-9);
2) E1301DB consisting of 98.7% oil extracted from *Fragaria x ananassa* and 1.3% natural tocopherol;
3) E1302D/A consisting of 99.8% oil extracted from Fragaria x ananassa and 0.2% antioxidant extract of rosemary (for example, the raw material *Rosmarinus Officinalis* Leaf Extract, CAS-No. 84604-14-8, EINECS-No. 283-291-9 dissolved in *Helianthus Annuus* Seed Oil, CAS-No. 8001-21-6, EINECS-No. 232-273-9);
4) E1302D/B consisting of 98.7% oil extracted from *Fragaria x ananassa* and 1.3% natural tocopherol;

All T0 sub-batches have the same quantity of peroxides as batches E1301D and E1302D, being precisely four aliquots. This step is useful to evaluate the possible addition of an antioxidant substance in the extract.

### EXAMPLE 3A

From batch SFR00122 consisting of 99.8% *Fragaria x ananassa* and 0.2% antioxidant extract of rosemary (e.g., the raw material Rosmarinus Officinalis Leaf Extract, CAS-No. 84604-14-8, EINECS-No. 283-291-9 dissolved in Helianthus Annuus Seed Oil, CAS-No. 8001-21-6, EINECS-No. 232-273-9) the extract is obtained as described in [3], wherein step a) envisages the starting biomass be subjected to air drying at a temperature above 40°C and uncontrolled for 48 hours. The extract was titrated to quantify the peroxide content. This titration resulted in a peroxide content of 141.4, i.e. at least 5 times the value obtained for the extract described in Example 3.

### REFERENCES.

1. E. Sikora, P. Michorczyk, M. Olszanska and J. Ogonowski. "Supercritical CO2 extract from strawberry seeds as a valuable component of mild cleansing compositions". International Journal of Cosmetic Science 37, 2015, 574-578
2. Lin T.-K., Zhong L., Santiago J.L. "Anti-inflammatory and skin barrier repair effects of topical application of some plant oils". Int. J. Mol. Sci. 19 (70), (2018)
3. Campalani C., Amadio E., Zanini S., Dall'Acqua S., Panozzo M., Ferrari S., De Nadai G., Francescato S., Selva M., Perosa A. "Supercritical CO2 as a green solvent for the circular economy: Extraction of fatty acids from fruit pomace". Journal of CO2 Utilization 41, (2020).
4. Turhan E. and Paydas Kargi S., "Strawberry Production in Turkey", Chronica Horticulturae, 47 (2), (2007) 18-20, ISSN 0578-039X.
5. Pilkington S. M., Watson R. E. B., Nicolaou A., & Rhodes L. E.. "Omega-3 polyunsaturated fatty acids: photoprotective macronutrients". Experimental dermatology, 20.7, (2011) 537-543.
6. Jie L., Kuniyoshi S., Ryuichiro K. "Anti-androgenic activity of fatty acids." Chem Biodivers, 6(4):503-12, (2009).
7. Azzouni F., Godoy A., Yun Li, Mohler J. "The 5 Alpha-Reductase Isozyme Family: A Review of Basic Biology and Their Role in Human Diseases." Advances in Urology. Article ID530121, 18, (2012).
8. Bernard F.-X., Barrault C., Deguercy A., De Wever B., Rosdy M. "Expression of type 1 5α-reductase and metabolism of testosterone in reconstructed human epidermis (SkinEthic®): a new model for screening skin-targeted androgen modulators." Int. J. Cosmet. Sci., 22(6):397, (2002).
9. Delos S., Carsol J.L., Ghazarossian E., Raynaud J.P., Martin P.M. "Testosterone metabolism in primary cultures of human prostate epithelial cells and fibroblasts." J Steroid Biochem Mol Biol, 55(3-4): 375-83, (1995)
10. Bayne E.K., Flanagan J., Instein M., Ayala J., Chang B., Azzolina B., Whiting D.A., Mumford R.A., Thiboutot D., Singer I.I., Harris G. "Immunohistochemical localization of types 1 and 2 5-alpha reductase in human scalp." Br J Dermatol Seo; 141(3): 481-91, (1999).
11. Higuchi R, Fockler C, Dollinger G, Watson R. "Kinetic PCR analysis: real-time monitoring of DNA amplification reactions." Biotechnology ; 11(9): 1026-30), (1993).
12. Higuchi R., Dollinger G., Walsh PS., Griffith R. "Simultaneous amplification and detection of specific DNA sequences." Biotechnology (N Y) 10(4):413-7, (1992

## Claims

1. Extract enriched in unsaturated fatty acids coming from processing waste in the production of foods based on *Fragaria vesca* and/or *Fragaria x ananassa,* obtained by supercritical extraction in CO2 and containing a quantity of peroxides less than 60 meq/kg of extract.

2. Extract according to claim 1, wherein the quantity of peroxides is less than 40 meq/ kg of extract, more preferably it is comprised between 15 and 35 meq/kg, preferably between 25 and 30 meq/kg of extract.

3. Process for preparing the extract according to any one of claims 1 or 2, comprising the following steps:
a) said food processing waste based on *Fragaria vesca* and/or *Fragaria x ananassa* is dried in an ionizing environment at a temperature comprised between 25°C and 45°C for a time comprised between 48 and 120 hours;
b) the dried product from step a) is supercritically extracted with CO2.

4. Process according to claim 3, wherein in step a) the drying is carried out in ionizing environment at temperature comprised between 30°C and 40°C for 48 hours and at 45°C for 72 hours.

5. Cosmetic composition comprising the extract according to claim 1 or 2 from processing waste in the production of foods based on *Fragaria vesca* and/or *Fragaria x ananassa.*

6. Cosmetic composition according to claim 5 being topically administrable.

7. Extract enriched in fatty acids from processing waste in the production of foods based on *Fragaria vesca* and/or *Fragaria x ananassa* according to claim 1 or 2 for use in the prevention and treatment of skin and hair disorders of inflammatory origin.

8. Extract for use according to claim 7, wherein said skin disorders of inflammatory origin are selected from: atopic dermatitis, psoriasis, sunburn, skin cancer, skin ageing, photosensitivity and photo ageing.

9. Extract enriched in fatty acids from processing waste in the production of foods based on *Fragaria vesca* and/or *Fragaria x ananassa* according to claim 1 or 2, for use in the prevention of skin and hair disorders associated with hormone imbalance.

10. Extract for use according to claim 9, wherein said hormone imbalance is the overproduction of dihydrotestosterone on skin and hair.

11. Extract for use according to claim 9 or 10, wherein said disorders are selected from: *acne vulgaris,* hirsutism, seborrhoea, *telogen effluvium* and androgenic alopecia.

12. Use of the extract enriched in fatty acids from processing waste in the production of foods based on *Fragaria vesca* and/or *Fragaria x ananassa* according to claim 1 or 2 in cosmetic compositions as emollient and humectant, to restore the moisture of the skin barrier layer.
